# EUROPÄISCHE PATENTSCHRIFT

(11) **EP 1 563 855 B1**
(45) Veröffentlichungstag und Bekanntmachung des Hinweises auf die Patenterteilung: **04.04.2007**
(21) Anmeldenummer: 05002336.5
(22) Anmeldetag: 04.02.2005
(51) Int. Cl.: A61L 15/28, A61L 15/44, A61L 15/60

(54) **Chitosanhaltige Wundauflagen**
Wound dressing comprising chitosan
Pansements comprenant du chitosane

(30) Priorität: 13.02.2004 DE 102004007115
(43) Veröffentlichungstag der Anmeldung: 17.08.2005
(73) Patentinhaber: Cognis IP Management GmbH, 40589 Düsseldorf (DE)
(72) Erfinder: Messinger, Horst, 52078 Aachen (DE)

(56) Entgegenhaltungen:
- EP-A- 0 415 183
- WO-A-01/41820
- WO-A-95/01808
- WO-A-96/13282
- WO-A-02/102276
- WO-A-20/04084961
- DE-A1- 19 712 699
- US-A- 4 532 134
- US-A- 5 658 582
- US-A- 5 836 970

## Beschreibung

### Gebiet der Erfindung

Die Erfindung befindet sich auf dem Gebiet der Pharmazie und betrifft Verbandsmaterialien, insbesondere mehrschichtige Wundauflagen, die als antimikrobiellen Wirkstoff Chitosan in einer Zwischenschicht enthalten.

### Stand der Technik

Die Anforderungen an Wundverbände und Wundauflagen sind vielfältig. Neben einer leichten Applizierbarkeit, Handhabung und sauberen schmerzfreien Entfernbarkeit sollen Wundauflagen sich der Wunde dennoch gut anpassen können und Schutz gegen äußere mechanische Einflüsse gewährleisten. Trotz eines guten Schutzes gegen Umwelteinflüsse muss eine ausreichende Luft- und Sauerstoffdurchlässigkeit ebenso wie die Wasserdampfdurchlässigkeit gegeben sein.
Die Heilung von Wunden wird durch ein empfindliches Feuchtigkeitsgleichgewicht beeinflusst. Einerseits muss das in der Wunde entstehende Sekret entfernt werden, so dass eine ausreichende Saugfähigkeit des abschließenden Wundverbandes notwendig ist, um Feuchtigkeit aufzunehmen und gegebenenfalls abzuführen, andererseits darf eine Wunde nicht austrocknen, da ein gewisses Maß an Feuchtigkeit für die Granulationsbildung wichtig ist. Außerdem würde bei einem ausgetrockneten Verband durch das Verkleben der aufliegenden Schicht mit der Wunde das frisch entstandene Granulationsgewebe wieder zerstört werden, wenn die Wundauflage entfernt wird.
In erster Linie sollen Wundauflagen jedoch gut physiologisch verträglich, hautfreundlich und möglichst keimfrei sein. Zur Reduzierung des Infektionsrisikos ist eine zusätzliche antimikrobielle Wirkung von Wundverbänden daher wünschenswert.

Die Verwendung von Chitosan im medizinischen und pharmazeutischen Bereich aufgrund seiner antimikrobiellen und entzündungshemmenden Eigenschaften ist lange bekannt. Besonders im Bereich der Wundverbände wird Chitosan antimikrobiellen Ausrüstung vielfach eingesetzt [Journal of Biomedical Materials Research, 2002 März, 59(3):438-449: Mi FL, Wu YB, Shyu SS, Schoung JY, Huang YB, Tsai YH, Hao JY. Control of wound infections using a bilayer chitosan wound dressing with sustainable antibiotic delivery.].
In der Europäischen Anmeldung **EP 1314410 A1** werden mehrschichtige Wundauflagen offenbart, die eine feuchtigkeitsabsorbierende Schicht aus gelbildenden Fasern, darunter auch Chitosan, mit einer nach außen abschließenden gut wasserdampfdurchlässigen Transmissionsschicht beschreibt. Chitosan ist jedoch mechanisch zu wenig stabil, um ohne stützende Flächengewebe sinnvoll in der Wundversorgung eingesetzt zu werden. Darüber hinaus hat es die Eigenschaft, nach Benetzung stark zu quellen und das resultierende Gel hat, insbesondere bei mechanischer Belastung, eine unzureichende Stabilität. Entsprechend muss eine Wunde, deren Exsudat das Chitosangel quellen ließ, durch regelmäßiges Ausspülen der losgelösten Chitosanreste nachbehandelt werden. Das ist zeit- und kostenaufwändig und verzögert zusätzlich die Wundheilung. Zur Erhöhung der Formstabilität sekretaufsaugender Polymere wird in der Europäischen Anmeldung **EP 0415183 A1** vorgeschlagen, quervernetzte Hydrogele einzusetzen. Die physiologische Verträglichkeit der als Vernetzer eingesetzten Alkoxysilylverbindungen ist bisher jedoch wenig untersucht. Eine Vernetzung der Polymere führt außerdem zu einer Abnahme der antimikrobiellen Eigenschaften des Chitosans. Die mechanischen Eigenschaften von Produkten, die ausschließlich aus aufgequollenem Chitosan bestehen, sind erfahrungsgemäß mangelhaft.
Auch Chitosanfolien, wie sie in der Internationalen Anmeldung **WO 95/01808 A1** beschrieben werden, sind nicht sehr reißfest und lassen sich trocken nur schlecht anspruchsvoll geformten Wundkavitäten anpassen. Auch in diesem Fall quillt die Chitosanfolie in feuchter Umgebung und bildet ein Gel, das in Wundauflagen Rückstände bildet, die regelmäßig durch Spülen der Wunde entfernt werden müssen.

Die Aufgabe der vorliegenden Erfindung hat somit darin bestanden, Wundverbände mit antimikrobieller Wirksamkeit und ausreichender Formstabilität zur Verfügung zu stellen, die sich geformten Kavitäten problemlos anpassen lassen, mechanischen Beanspruchungen standhalten, das die Wundheilung fördernde Feuchtgleichgewicht aufrechterhalten und sauber und schmerzfrei zu entfernen sind.

### Beschreibung der Erfindung

Gegenstand der Erfindung sind Wundauflagen, umfassend
(a) eine biokompatible durchlässige Schicht zur Auflage auf der Wunde, daran anschließend
(b) eine chitosanhaltige Zwischenschicht, in der Chitosan in Form eines Granulates, einer Folie oder einer porösen Matrix vorliegt und
(c) mindestens eine luft- und sauerstoffdurchlässige Schicht, die als Stützgewebe und Abschluss für die chitosanhaltige Zwischenschicht dient.

Durch ein flexibles Flächengewebe wird das Chitosangel vor direktem Kontakt mit dem Wundgewebe geschützt. Eine Sandwichstruktur des Flächengewebes ist bevorzugt, wobei die innere Lage aus flächigem oder granuliertem Chitosan besteht. Das Exsudat durchdringt das äußere Stützgewebe und lässt das im Inneren befindliche Chitosan quellen, wo ebenfalls die antimikrobielle Wirkung des Chitosans eintritt. Dem Chitosan gegebenenfalls zugesetzte Wirkstoffe werden kontinuierlich freigesetzt und können die Wundheilung weiter fördern. Der Verbandswechsel ist einfach durch Wechsel der Wundauflage durchzuführen, ein Spül- oder Reinigungsvorgang entfällt.

Es wurde gefunden, dass die so aufgebauten Wundauflagen in zweifacher Hinsicht eine gute antimikrobielle Wirksamkeit aufweisen. Einerseits wirken sich antimikrobielle Effekte innerhalb der Wundauflage positiv aus: So wird keimhaltiges Wundsekret von der Wundauflage gut aufgenommen, aber dennoch führt die antimikrobielle Ausrüstung zu einer verminderten Keimbelastung der Wundauflage, so dass ein häufiger Verbandswechsel vermieden werden kann. Andererseits wirken sich die antimikrobiellen Eigenschaften auch auf die direkte Wundoberfläche aus: Denn es diffundieren geringe Mengen gelöster Chitosanmoleküle durch die durchlässige Membran in die Wunde und bewirken hier einen antimikrobiellen Effekt sowie eine verbesserte Wundheilung und Geweberegeneration ohne gelartige Rückstände zu hinterlassen, die nach Abnehmen des Verbandes unter Schmerzen entfernt werden müssen.
Daneben zeichnet sich die erfindungsgemäße Wundauflage durch eine gute Formstabilität aus, erlaubt es aber dennoch auch tiefer geformte Kavitäten, beispielsweise bei Decubitus, mit dem heilungsfördernden Verband flächig auszukleiden. Durch die aufsaugende Wirkung des Chitosangels wird die Wunde von Exsudat gereinigt. Obgleich das Wundsekret schnell und nachhaltig durch die biokompatible durchlässige Schicht in die chitosanhaltige Zwischenschicht abgeführt wird, trocknet die Wunde aufgrund der gequollenen feuchthaltenden Chitosan-Gelschicht nicht aus und das die Wundheilung fördernde Feuchtegleichgewicht bleibt erhalten.

### Chitosane

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

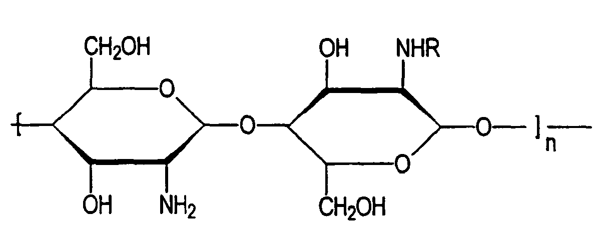

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus der französischen Patentanmeldung FR 2701266 A bekannt. Vorzugsweise werden solche Typen eingesetzt, wie sie in den deutschen Patentanmeldungen **DE 4442987 A1** und **DE 19537001 A1** (Henkel) offenbart werden und die ein durchschnittliches Molekulargewicht von 10.000 bis 5.000 000 Dalton, insbesondere 10.000 bis 500.000 bzw. 800.000 bis 1.200.000 Dalton aufweisen und/oder eine Viskosität nach Brookfield (1 Gew.-%ig in Glycolsäure) unterhalb von 30.000 mPas, einen Deacetylierungsgrad im Bereich von 80 bis 88 % und einem Aschegehalt von weniger als 0,3 Gew.% besitzen. Üblicherweise werden Chitosane mit einem mittleren Molekulargewicht von 10.000 bis 5.000.000 Dalton eingesetzt, in einer bevorzugten Ausführung werden Chitosane mit einem durchschnittlichen Molekulargewicht von 30.000 bis 1.000.000 Dalton eingesetzt, weiterhin bevorzugt sind Chitosane mit einem Molekulargewicht von 40.000 bis 500.000 Dalton, besonders bevorzugt sind aber Chitosane mit einem Molekulargewicht von 50.000 bis 100.000 Dalton.

Die auf diesem Wege erhältlichen Chitosane stellen Chitosane mit höherem Molekulargewicht dar. Speziell eingesetzt werden auch sogenannte Oligoglucosamine, bei denen es sich um Chitosane mit geringem Molekulargewicht handelt. Diese werden in einem zweiten Schritt unter dem Einfluss von Säuren weiter abgebaut und die Spaltprodukte, also die Oligoglucosamine, die jetzt ein Molekulargewicht von 500 bis 5.000 und vorzugsweise 800 bis 1.500 aufweisen einer Membranfiltration unterworfen, um sie von Verunreinigungen und insbesondere von Salzen zu befreien. Diese Oligochitosane haben neben der antimikrobiellen Wirksamkeit zusätzlich eine regenerative und wachstumsstimulierende Wirkung, sowie anti-inflammatorische Eigenschaften und bieten sich somit bevorzugt für die Anwendung in Wundauflagen an.

Neben den Chitosanen als typischen kationischen Biopolymeren kommen im Sinne der Erfindung auch derivatisierte Chitosane in Betracht, bei denen der kationische Charakter durch die Derivatisierung erhalten bleibt.
Um ein Durchdringen der porösen direkten Wundauflagenschicht (a) mit feinteiligen Chitosanpartikeln zu verhindern, wird das Chitosan als Granulat, poröse Matrix oder Film in der Zwischenschicht (b) eingesetzt. Die Herstellung chitosanhaltiger Filme kann beispielsweise wie in WO 95/01808 A1 beschrieben erfolgen.

### Herstellung einer porösen Matrix oder eines Granulates

Zur Herstellung eines Granulates oder einer porösen Matrix werden die Chitosane in wässerigen Mineralsäuren oder wässerigen organischen Carbonsäuren gelöst oder suspendiert. Die Suspensionen der Chitosane enthalten in der Regel gelöste Anteile an Chitosanen. Als Mineralsäuren sind geeignet Salzsäure, Phosphorsäure, Salpetersäure sowie Schwefelsäure, als organische Carbonsäuren seien genannt: Ameisensäure, Milchsäure, Propionsäure, Maleinsäure, Brenztraubensäure, Glykolsäure, Bernsteinsäure, Essigsäure, Zitronensäure, Weinsäure sowie Adipinsäure. Besonders bevorzugt sind Salzsäure, Milchsäure sowie Glykolsäure. Es werden die Mengen an Säure eingesetzt, die zu einer teilweisen bis vollständigen Lösung des Chitosans benötigt werden. Üblicherweise sind dies 10⁻⁴ bis 10⁻² mol Säuregruppen pro g Chitosan, insbesondere 1 - 3 x 10⁻³ mol Säuregruppen / g Chitosan.

In der Regel wird eine 0,1 bis 15 Gew.-%ige wässerige Lösung bzw. Suspension eingesetzt, bevorzugt ist eine 0,5 bis 10 Gew.-%ige, insbesondere eine 1,0 bis 5,0 Gew.-% und insbesondere eine 1,5 bis 2,5 Gew.-%ige wässerige Lösung bzw. Suspension. Handelt es sich um eine Suspension, die in der Regel gelöste Anteile enthält, so kann es vorteilhaft sein, die Suspension zu homogenisieren. Die wässrige Lösung bzw. homogenisierte Suspension hat in der Regel einen pH-Wert im Bereich von 1,0 bis 7,5, insbesondere im Bereich von 4,5 bis 6,5.
Zur gezielten Fällung des Polymers aus der Lösung oder Suspension wird nun ein Fällungsmittel eingesetzt.

Als Fällungsmittel im Sinne der Erfindung sind prinzipiell alle Stoffe geeignet, die den pH-Wert der wässerigen Lösung bzw. homogenisierte Suspension erhöhen. Geeignet sind hierfür wässerige Lösungen von Carbonaten, Hydrogencarbonaten, Hydrogenphosphaten und Hydroxiden der Alkali- und Erdalkalimetalle, Ammoniak und organischen Stickstoffbasen. Als organische Stickstoffbasen sind beispielsweise Triethylamin, Triethanolamin oder Tetraalkylammoniumhydroxide geeignet. Die wässerigen Lösungen der Fällungsmittel werden üblicherweise in einer Konzentration von 5 bis 20 Gew.-%, insbesondere von 7 bis 16 Gew.-%, eingesetzt. In einer bevorzugten Ausführungsform der vorliegenden Erfmdung wird als Fällungsmittel eine wässerige Natriumhydrogencarbonatlösung eingesetzt, insbesondere eine 7 bis 16 Gew.-%, bevorzugt eine 7 bis 9 Gew.-% wässerige Natriumhydrogencarbonatlösung.
Durch die Behandlung mit dem Fällungsmittel wird der pH-Wert der wässerigen Lösung bzw. homogenisierten Suspension der Biopolymere in der Regel auf einen pH-Wert von 5,0 bis 14 eingestellt, insbesondere auf einen pH-Wert von 7,0 bis 8,5.
Nachfolgend wird das so behandelte Produkt entwässert. Geeignete Methoden der Entwässerung sind beispielsweise die Lufttrocknung, die Vakuumtrocknung, insbesondere bei Temperaturen von 20 bis 100 °C oder über 100 °C, sowie die Gefriertrocknung.

Gegebenenfalls ist der Entwässerung ein Einfrierschritt voranzustellen. Dies ist insbesondere in der Kombination mit der Gefriertrocknung vorteilhaft. Die vorliegende Erfindung umfasst die Erkenntnis, dass die durch das Ausfällen der Fasern erzeugte Struktur, die durch den Einfriervorgang fixiert wird, bei der Entwässerung in Form der Gefriertrocknung im wesentlichen erhalten bleibt. Es wird somit eine mechanisch relativ stabile Form des Chitosans ohne die Anwendung von Vernetzern erhalten. Hierzu wird die auf die gewünschte Viskosität eingestellte und mit Fällungsmittel vermischte Suspension bei Temperaturen unterhalb des Gefrierpunkts eingefroren. Die Art und Weise der Durchführung des Einfriervorgangs hat einen großen Einfluß auf das Erscheinungsbild und die Struktur der nach der Gefriertrocknung entstehenden porösen Matrix. So gilt z.B. je schneller der Einfriervorgang ist, desto feinporiger und gleichmäßiger wird die nach der Gefriertrocknung entstehende Matrix.
Verfolgt man die Verwendung von Chitosangranulaten wird die nach Trocknung erzeugte Matrix durch die üblichen Methoden der Zerkleinerung fester Güter auf die gewünschte Größe zerkleinert.
Nicht ausgeschlossen ist auch die Herstellung eines Granulates auf dem Wege der aufbauenden Granulierung durch Einsatz von Chitosan in Verbindung mit den üblichen Granulierungshilfsstoffen, jedoch findet in Wundauflagen bevorzugt das reinere Chitosangranulat unter Anwendung von möglichst wenig Hilfsstoffen Anwendung.

Die Herstellung über Gefriertrocknung bietet sich bevorzugt an, wenn weitere Wirkstoffe in das Chitosangranulat oder die Chitosanmatrix eingebettet werden sollten, die temperaturempfindlich sind. Die Zugabe weiterer Hilfs- und Wirkstoffe kann sowohl vor der Zugabe des Fällungsmittels als auch zusammen mit dem Fällungsmittel erfolgen. Bei Anwendung der Granulatform belädt man auch die Zubereitungen nach der Entwässerung mit Hilfs- und Wirkstoffen. Hierbei werden beispielsweise kosmetische und pharmazeutische Wirkstoffe oder Aromastoffe mit speziellen Techniken auf die fertige, trockene Zubereitung nach der Gefriertrocknung aufgebracht. Dazu wird der Wirkstoff in einem geeigneten Lösungsmittel gelöst, auf den nach der Gefriertrocknung entstehenden Schwamm, der in dieser Ausführungsform als Trägermaterial fungiert, aufgebracht und das Lösungsmittel dann schonend entfernt. Geeignete Lösungsmittel sind beispielsweise überkritisches CO₂ oder unpolare oder polare organische Lösungsmittel, wie beispielsweise Hexan, Ethanol oder Isopropanol. Bevorzugt wird das trockene Aufziehen feinteiliger Wirkstoffe auf das fertige Granulat.

### Weitere Wirkstoffe

Weitere Wirkstoffe, die die Wundheilung beschleunigen und ebenfalls in die erfindungsgemäße Wundauflage vorzugsweise in die Zwischenschicht inkorporiert werden können, sind beispielsweise Pflanzenextrakte wie Aloe vera, Antibiotika, Antiseptika, Antimykotika, Analgetika, Antihistaminika, Enzyminhibitoren, Glucocorticoide, Vitamine, Virustatika, Wachstumsfaktoren, Steroide, Enzyme oder Hormone.
Bei der Anwendung weiterer Wirkstoffe in der Wundauflage bietet es sich an, die Abgabe der Wirkstoffe über eine kontrollierte Freisetzung zu steuern. Dies kann durch eine die Wirkstoffabgabe steuernde Membran erfolgen, oder durch Verwendung ausgewählter Molekulargewichte des Chitosans, welches dann auch als Wirkstoffreservoir dient. Gegebenenfalls können weitere Polymere in die Zwischenschicht zur Aufgabe der Freisetzungssteuerung beitragen.

### Aufbau der Wundauflage

Die erste mit der Wunde direkt in Kontakt kommende biokompatible durchlässige Schicht (a) dient der Abtrennung der antimikrobiellen und wundsekretaufsaugenden chitosanhaltigen Schicht von der Wunde. Sie muss für Exsudat durchlässig sein, es aber möglichst selber nicht aufsaugen und speichern, da ansonsten die Gefahr eines mikrobiellen Befalls besteht. Sie sorgt dafür, dass die Formstabilität der chitosanhaltigen Zwischenschicht auch nach Aufsaugen des Wundsekretes noch erhalten bleibt und keine Verunreinigung durch gequollene Partikel in der Wunde entstehen, der Wundverband kann ohne Rückstände entfernt werden.
Als durchlässige Schicht (a) eignen sich Vliese oder poröse Membranen aus biokompatiblen Materialien wie Seide, Polyvinylalkohol, Polycarbonate, Celluloseester wie Celluloseacetat, Cellulosenitrat, regenerierte Cellulose, sowie Polyolefine wie Polypropylen, Polyethylen oder Copolymere aus Ethylen oder Propylen mit Butadien. Weiterhin werden auch Polyesterfasern, beispielsweise Polyethylenterephthalatfasern, verwendet. Insbesondere geeignet sind auch Fasern, die aus zwei oder mehr Komponenten bestehen, beispielsweise Polyester-Copolyesterfasern oder Polypropylen-Polyethylenfasern und Polyamide.
Werden als Auflageschicht Vliesstoffe verwandt, so können diese nach allen im Stand der Technik bekannten Verfahren der Vliesherstellung, wie sie beispielsweise in **Ullmann's Encyclopedia of Industrial Chemistry, Vol. A 17, VCH Weinheim 1994, Seiten 572 - 581,** beschrieben werden, hergestellt werden. Bevorzugt sind dabei Vliese, die entweder nach dem sogenannte "dry laid"- oder dem Spinnvlies- oder spunbond-Verfahren hergestellt wurden. Das "dry laid"-Verfahren geht von Stapelfasern aus, die üblicherweise durch Kardieren in Einzelfasern getrennt und anschließend unter Einsatz eines aerodynamischen oder hydrodynamischen Verfahrens zum unverfestigten Vliesstoff zusammengelegt werden.
Dieser wird dann beispielsweise durch eine thermische Behandlung zum fertigen Vlies verbunden (das sogenannte "thermobonding"). Dabei werden die synthetischen Fasern entweder soweit erwärmt, daß deren Oberfläche schmilzt und die Einzelfasern an den Kontakstellen miteinander verbunden werden, oder die Fasern werden mit einem Additiv überzogen, welches bei der Wärmebehandlung schmilzt und so die einzelnen Fasern miteinander verbindet. Durch Abkühlung wird die Verbindung fixiert. Neben diesem Verfahren sind natürlich auch alle anderen Verfahren geeignet, die im Stand der Technik zum Verbinden von Vliesstoffen eingesetzt werden. Die Spinnvliesbildung geht dagegen von einzelnen Filamenten aus, die nach dem Schmelzspinnverfahren aus extrudierten Polymeren gebildet werden, welche unter hohem Druck durch Spinndüsen gedrückt werden. Die aus den Spinndüsen austretenden Filamente werden gebündelt, gestreckt und zu einem Vlies abgelegt, welches üblicherweise durch "thermobonding" verfestigt wird.
Bei der Verwendung von porösen Membranen werden vorzugsweise Celluloseester eingesetzt. Diese werden zur Herstellung der Membranen in einem leicht flüchtigen Lösungsmittel gelöst und mit einem geringen Anteil eines schwerer flüchtigen Nichtlösungsmittels versetzt. Diese Mischung wird in dünner Schicht auf ein endloses poliertes Band aufgestrichen und belüftet. Dabei verdunstet das Lösungsmittel, das schwerer flüchtige Nichtlösungsmittel reichert sich an, bis schließlich ein Gel des makromolekularen Stoffes ausfällt. Durch Trocknung erhält man die Membranfolie, deren Porenstruktur von der Konzentration der Ausgangslösung und von den Verdunstungsbedingungen abhängig ist. Bei der Herstellung von porösen Polycarbonatmembranen wird eine porenfreie sehr dünne Folie aus Polycarbonaten einem Neutronenbeschuss ausgesetzt und anschließend durch ein alkalische Ätzbad geführt. An den Stellen des Neutronendurchgangs werden Carbonatreste gelockert und diese im Ätzbad herausgelöst.
Insbesondere bei porösen Membranen wie Celluloseestern oder Polycarbonatmembranen kann durch die Herstellungsweise eine genau definierte Porengröße und Porosität gezielt eingestellt werden, so dass sich diese Materialien besonders für Wundauflagen eignen, die zusätzliche Wirkstoffe enthalten und eine gesteuerte Freisetzung erfordern.

Die chitosanhaltige Zwischenschicht (b), in der Chitosan in Form eines Granulates, einer Folie oder einer porösen Matrix vorliegt, kann gegebenenfalls weitere pharmazeutische Wirkstoffe oder Pflanzenextrakte enthalten. Diesen können durch Mischungen des Chitosangranulates mit anderen Granulaten, Pulvern oder Zubereitungen hergestellt werden oder direkt bei der Herstellung des Chitosangranulates, der Chitosanmatrix oder Chitosanfolie zugefügt werden.
Neben den Chitosanen kann die Zwischenschicht noch weitere quellfähige Polymere enthalten. Da Chitosan ein kationisches Biopolymer darstellt, ist es auch möglich durch Kombination mit anionischen Polymeren und dadurch Bildung eines höher viskosen quellenden Gels, die Festigkeit und Formstabilität der Zwischenschicht weiterhin zu erhöhen. Entsprechende Polymere für die Bildung sogenannter Polyanion-Polykation-Komplexe sind in der Europäischen Patentanmeldung **EP1264014 A1** beschrieben.

Generell sollte der Anteil an Chitosanen in der Zwischenschicht jedoch mindestens 10 Gew. %, bevorzugt mindestens 50 Gew.%, besonders bevorzugt mindestens 80 Gew. % bezogen auf das Material in der Zwischenschicht betragen.
In einer bevorzugten Ausführungsform ist die chitosanhaltige Zwischenschicht (b) durch Kammern unterteilt. Dadurch wird die Formstabilität zusätzlich erhöht und es ist möglich, tiefere Kavitäten durch die Wundauflage auszufüllen, ohne dass die Struktur des Wundverbandes zerstört wird. Auch ein einfaches und sauberes Entfernen wird somit ermöglicht. Die Kammerstruktur kann durch Verfahren wie Abnähen oder Thermo-Bonding erreicht werden. Es wird somit pro Flächeneinheit immer dieselbe Menge Chitosan zur Verfügung gestellt. Insbesondere bei Systemen, die zusätzliche Wirkstoffe enthalten oder als topische Applikationsform dienen ist die durch die Kammerung unterstützte gleichmäßige Dosierung vorteilhaft.

Die chitosanhaltige Zwischenschicht wird durch mindestens eine luft- und sauerstoffdurchlässige Schicht (c), die als Stützgewebe und Abschluss für die chitosanhaltige Zwischenschicht dient, umschlossen. Diese besteht aus einem gut luftdurchlässigen Gewebe, das das Chitosangranulat und die Feuchtigkeit wirksam zurückhält. Sie sollte feuchtigkeitsundurchlässig aber gegebenenfalls wasserdampfdurchlässig sein. Dieses kann auch mit einer geeigneten Kombination von Materialschichten realisiert werden.
Als Materialien für diese Schicht eignen sich vor allem Polyester, aber auch wie die bereits teilweise unter der Wundkontaktschicht (a) aufgezählten anderen hautverträglichen Kunststoffe, wie Polyvinylchlorid, Ethylen- Vinylacetat-Copolymere, Polyvinylacetat, Polyethylen, Polypropylen, Polyurethane oder Cellulosederivate und viele andere mehr. Diese Deckschicht kann weiterhin überzogen werden, so dass sich durch Bedampfung mit Metallen, insbesondere Aluminium, oder anderen diffusions- sperrenden Zusatzstoffen wie beispielsweise Siliciumdioxid eine weitere Schicht ergibt.

### Gewerbliche Anwendbarkeit

Die erfindungsgemäßen antimikrobiell wirksamen Wundverbände zeichnen sich durch hohe Hautverträglichkeit und ein hohes Flüssigkeitsaufnahmevermögen ohne Verlust der notwendigen Formstabilität aus. Ein weiterer Gegenstand der Erfindung betrifft daher die Verwendung der erfindungsgemäßen Zubereitungen zur Wundbehandlung als Wundtampons, Wundverbände, Brandwundverbände, wirkstoffabgebende Verbände, Vliese, transdermale therapeutische Systeme und dermale Drug Carrier. Dabei können die erfindungsgemäßen Zubereitungen mit verschiedenen topisch anwendbaren pharmazeutischen Wirkstoffen und Formulierungen beladen werden. Die erfindungsgemässen Wundauflagen können auch in Form eines Pflasters mit einer klebefähigen Randfläche vorliegen und ermöglichen so eine einfache, bequeme und schnelle Handhabung. Eine Sterilisation des Verbandmaterials kann auf übliche Art und Weise - vorzugsweise durch ionisierende Strahlung - erfolgen.

### Beispiele

### Beispiel 1a

### Herstellung des Chitosangranulats:

Eine Suspension aus 2 kg Chitosan (Hydagen ®CMFP, Henkel KGaA), 98 kg Wasser und 0,346 kg L-(+)-Milchsäure wurden mittels einer Kolloidmühle solange bei einer Temperatur von 40 °C homogenisiert, bis eine Viskosität von 23000 mPas erreicht wurde. Danach wurde die Suspension auf 10 °C abgekühlt und im Vakuum entgast. Jeweils 9 kg der Suspension wurden mit 360 g einer wässerigen Lösung von Natriumhydrogencarbonat (= 8,05 Gew.-%ige wässerige Natriumhydrogencarbonatlösung) für 2 Minuten gemischt und anschließend in Formen gegossen. Die Schichtdicke der Suspension in der Form betrug 22 mm. Nach einer Ruhezeit von 3h wurde die Suspension eingefroren und anschließend die gefrorenen Platten bei 80°C und 1 mbar gefriergetrocknet.

Die getrockneten Blöcke wurden anschließend mit der Schlagstiftmühle zerkleinert. Durch Siebung wurde eine Fraktion mit einer durchschnittlichen Teilchengröße von 1 bis 2 mm abgetrennt.

### Beispiel 1b

Die Herstellung des Chitosangranulats erfolgte entsprechend Beispiel 1a wobei als Chitosan ein Oligoglucosamins mit einem mittleren Molekulargewicht von 500 eingesetzt wurde, welches durch sauren Abbau von Chitosan (Hydagen® DCMF, Cognis Deutschland GmbH & Co. KG) hergestellt worden war.

### Herstellung der Wundauflage:

### Beispiel 2a

Auf einem thermobonded Polyamidvlies wurde das Chitosangranulat des Beispiels 1a so verteilt, dass pro 1 cm² Flächeneinheit 1 ± 0,3 g Granulat aufgetragen wurden und nachfolgend mit einer atmungsaktiven wasserundurchlässigen Polyesterschicht abgedeckt wurden. Der mehrlagige Wundverband wurde durch Abnähen in Kammern mit einer Breite und Länge von ca. 1,3 cm unterteilt.

### Beispiel 2b

Beispiel 2a wurde wiederholt mit dem Granulat aus Beispiel 1b.

### Beispiel 3

Beispiel 2a wurde wiederholt, wobei das Antibiotikum Silbersulfadiazin dem Granulat 1 a durch Mischen des feinteiligen Pulvers mit dem Granulat in einer Menge von 10 mg bezogen auf die Menge 1 g Chitosangranulat zugefügt worden war.

### Beispiel 4

Beispiel 2a wurde wiederholt, wobei Aloe vera (Alovera Konzentrat Pulver, Aloecare, 53501 Grafschaft) dem Granulat 1 a durch Mischen des feinteiligen Pulvers mit dem Granulat in einer Menge von 3 Gew. % bezogen auf die Menge Chitosangranulat zugefügt worden war.

Die in den Beispielen beschriebenen Wundauflagen wurden mit definierten Mengen Wasser als Exsudatsimulans getränkt. Im Gegensatz zu den mit der auf das Flächengewicht bezogen gleichen Menge Wasser getränkten Chitosanfolien (Polymoist Marine, Cognis Deutschland GmbH & Co KG) behielten die beschriebenen Wundauflagen ihre volle mechanische Stabilität und konnten in der gewünschten Form am menschlichen Körper fixiert werden, ohne dass es zu einem Austritt von Chitosan durch das biokompatible Trennmaterial kam, das eine Wunde hätte verunreinigen können. Die reinen Polymoist Chitosanfolien waren dagegen in gequollenem Zustand weder mechanisch belastbar noch dauerhaft fixierbar. Die resultierenden Chitosan-Bruchstücke sind aus Wunden nur schwer zu entfernen und würden den Heilungsprozess nicht optimal ablaufen lassen.

## Patentansprüche

1. Wundauflage, umfassend
a. eine biokompatible durchlässige Schicht zur Auflage auf der Wunde, daran anschließend
b. eine chitosanhaltige Zwischenschicht, in der Chitosan in Form eines Granulates, einer Folie oder einer porösen Matrix vorliegt und
c. mindestens eine luft- und sauerstoffdurchlässige Schicht, die als Stützgewebe und Abschluss für die chitosanhaltige Zwischenschicht dient.

2. Wundauflage gemäß Anspruch 1, **dadurch gekennzeichnet, dass** die chitosanhaltige Zwischenschicht (b) durch Kammern unterteilt ist.

3. Wundauflage gemäß den Ansprüchen 1 und/oder 2, **dadurch gekennzeichnet, dass** die chitosanhaltige Zwischenschicht (b) weitere Wirkstoffe enthält.

4. Wundauflage gemäß den Ansprüchen 1 bis 3, **dadurch gekennzeichnet, dass** die chitosanhaltige Zwischenschicht (b) als Chitosane kationisch derivatisierte Chitosane enthält.

5. Wundauflage gemäß den Ansprüchen 1 bis 4, **dadurch gekennzeichnet, dass** die chitosanhaltige Zwischenschicht (b) Chitosane mit einem mittleren Molekulargewicht von 10.000 bis 5.000.000 enthält.

6. Wundauflage gemäß den Ansprüchen 1 bis 5, **dadurch gekennzeichnet, dass** die chitosanhaltige Zwischenschicht (b) Chitosane mit einem mittleren Molekulargewicht von 500 bis 5.000 enthält.

7. Wundauflage gemäß den Ansprüchen 1 bis 6, **dadurch gekennzeichnet, dass** die biokompatible durchlässige Schicht (a), die direkten Kontakt zur Wunde hat, eine kontrollierte Wirkstofffreisetzung steuert.

8. Wundauflage gemäß den Ansprüchen 1 bis 7, **dadurch gekennzeichnet, dass** sie in Form eines Pflasters mit klebefähigen Randflächen vorliegt.

9. Wundauflagen gemäß den Ansprüchen 1 bis 8 in der Verwendung zur Wundbehandlung und beschleunigten Wundheilung.

## Claims

1. Wound dressing comprising
(a) a biocompatible, permeable layer for application to the wound, then
(b) a chitosan-containing interlayer in which chitosan is present in the form of granules, a film or a porous matrix and
(c) at least one air- and oxygen-permeable layer acting as a supporting fabric and as a seal for the chitosan-containing interlayer.

2. Wound dressing as claimed in claim 1, **characterized in that** the chitosan-containing interlayer (b) is divided into compartments.

3. Wound dressing as claimed in claims 1 and/or 2, **characterized in that** the chitosan-containing interlayer (b) contains other active principles.

4. Wound dressing as claimed in claims 1 to 3, **characterized in that** the chitosan-containing interlayer (b) contains cationically derivatized chitosans as the chitosans.

5. Wound dressing as claimed in claims 1 to 4, **characterized in that** the chitosan-containing interlayer (b) contains chitosans with an average molecular weight of 10,000 to 5,000,000.

6. Wound dressing as claimed in claims 1 to 5, **characterized in that** the chitosan-containing interlayer (b) contains chitosans with an average molecular weight of 500 to 5,000.

7. Wound dressing as claimed in claims 1 to 6, **characterized in that** the biocompatible permeable layer (a) which is in direct contact with the wound controls the release of active components.

8. Wound dressing as claimed in claims 1 to 7, **characterized in that** it is in the form of a plaster with adhesive margins.

9. Wound dressings as claimed in claims 1 to 8 used for wound treatment and accelerated wound healing.

## Revendications

1. Pansement comprenant :
a) une couche biocompatible perméable destinée à être appliquée sur la plaie, à laquelle on associe
b) une couche intermédiaire contenant du chitosane, dans laquelle le chitosane se présente sous la forme de granulés, d'un film ou d'une matrice poreuse, et
c) au moins une couche perméable à l'air et à l'oxygène, servant de tissu de support et de finition pour la couche intermédiaire contenant du chitosane.

2. Pansement selon les revendications 1,
**caractérisé en ce que**
la couche intermédiaire contenant du chitosane (b) est divisée en compartiments.

3. Pansement selon les revendications 1 et/ou 2,
**caractérisé en ce que**
la couche intermédiaire contenant du chitosane (b) contient des principes actifs supplémentaires.

4. Pansement selon les revendications 1 à 3,
**caractérisé en ce que**
la couche intermédiaire contenant du chitosane (b) contient, comme chitosanes, des dérivés cationiques de chitosanes.

5. Pansement selon les revendications 1 à 4,
**caractérisé en ce que**
la couche intermédiaire contenant du chitosane (b) contient des chitosanes de poids moléculaire moyen de 10 000 à 5 000 000.

6. Pansement selon les revendications 1 à 5,
**caractérisé en ce que**
la couche intermédiaire contenant du chitosane (b) contient des chitosanes de poids moléculaire moyen de 500 à 5 000.

7. Pansement selon les revendications 1 à 6,
**caractérisé en ce que**
la couche biocompatible perméable (a) en contact direct avec la plaie commande une libération régulée de principe actif.

8. Pansement selon les revendications 1 à 7,
**caractérisé en ce qu'**
il est sous forme d'un tampon à bords adhésifs.

9. Pansement selon les revendications 1 à 8, destiné à être utilisé pour le traitement des plaies et l'accélération de la cicatrisation.
